(19) Europäisches Patentamt / European Patent Office / Office européen des brevets

(11) **EP 4 496 511 B1**

(12) **EUROPEAN PATENT SPECIFICATION**

(45) Date of publication and mention
of the grant of the patent:
**20.05.2026  Bulletin 2026/21**

(21) Application number: **23710908.7**

(22) Date of filing: **15.03.2023**

(51) International Patent Classification (IPC):
**A61B 5/11** (2006.01)   **A61B 5/00** (2006.01)
**G01C 22/00** (2006.01)   **A61B 5/024** (2006.01)

(52) Cooperative Patent Classification (CPC):
**A61B 5/7221; A61B 5/02438; A61B 5/1118;
A61B 5/6802; A61B 5/681; A61B 5/6898;
G01C 22/006**

(86) International application number:
**PCT/EP2023/056649**

(87) International publication number:
**WO 2023/180145 (28.09.2023 Gazette 2023/39)**

(54) **METHOD, DEVICE AND SYSTEM FOR ASSESSING RELIABILITY OF ESTIMATED METRICS RELATING TO A PHYSIOLOGICAL STATE OF A USER**

VERFAHREN, VORRICHTUNG UND SYSTEM ZUR BEWERTUNG DER ZUVERLÄSSIGKEIT VON GESCHÄTZTEN METRIKEN IN BEZUG AUF EINEN PHYSIOLOGISCHEN ZUSTAND EINES BENUTZERS

PROCÉDÉ, DISPOSITIF ET SYSTÈME D'ÉVALUATION DE LA FIABILITÉ DES MESURES ESTIMÉES RELATIVES À UN ÉTAT PHYSIOLOGIQUE D'UN UTILISATEUR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **22.03.2022   EP 22163495**

(43) Date of publication of application:
**29.01.2025   Bulletin 2025/05**

(73) Proprietor: **Koninklijke Philips N.V.
5656 AE Eindhoven (NL)**

(72) Inventors:
• **PAPINI, Gabriele**
  **5656 AG Eindhoven (NL)**
• **COX, Lieke, Gertruda, Elisabeth**
  **5656 AG Eindhoven (NL)**
• **MUESCH, Guido, Josef**
  **5656AG Eindhoven (NL)**

(74) Representative: **Philips Intellectual Property & Standards
High Tech Campus 52
5656 AG Eindhoven (NL)**

(56) References cited:
DE-A1- 102015 004 304    US-A1- 2013 080 255
US-A1- 2013 138 007      US-A1- 2015 099 945

**Description**

FIELD OF THE INVENTION

The present invention relates to a method, device and system for assessing reliability of estimated metrics relating to a physiological state of a user.

BACKGROUND OF THE INVENTION

Monitoring activity and vital metrics continuously, unobtrusively, and remotely is becoming increasingly popular to improve patient care, reduce hospital stay and boost patient outcomes. Ambulatory speed, i.e. walking speed, is considered the sixth vital sign and can be used to assess the physical performance of patients in clinical practice and determine frailty and associated health risks. Activity level (e.g., in the form of step counting) can assess patients' overall level of mobility in hospital and help contextualize physiological response to activity. All these metrics are becoming particularly relevant in ambulatory patient monitoring devices to augment a hospital's decision-making process, for example, related to discharge readiness, which so far is in most cases relying on subjective input from nurses on activity and patient condition.

Several algorithms for estimation of physiological and anatomical metrics, particularly ambulatory metrics, exist. Most of these algorithms use features of acceleration and cardiovascular signals (e.g., obtained by on-body accelerometers, photoplethysmography (PPG), and electrocardiography (ECG)) and data analysis techniques (e.g., linear regression and machine learning) to estimate the metrics (e.g. walking speed, step counting, and walking economy).

US 2013/0138007 A1 describes a technique of measuring a heart rate of a user. The disclosed heartbeat measuring device comprises an information storage unit that stores unique information regarding a heart rate unique to a user or information regarding a detected heartbeat therein and a determination unit that determines whether or not a heartbeat measurement state of the user is normal on the basis of the unique information or the information regarding a heartbeat, stored in the information storage unit.

Further methods for estimating physiological or anatomical metrics are described in US 2015/0099945 A1 and US 2013/0080255 A1.

As for every algorithm, the estimation might deviate from the actual value due to unforeseen data or inherent limitations of the features and data analysis techniques used. In fact, ambulatory metrics estimation is always characterized by a certain amount of error. However, improving the estimation results of the mentioned estimation methods might not always be possible, and it does not guarantee that estimation errors will not occur on new data. Unfortunately, these estimation errors might lead to a wrong assessment of a patient's status. Apart from said severe consequences in clinical decisions, wrong estimations of a user's physiological or anatomical metrics may lead to a wrong assessment of the user's fitness level and thus to inappropriate training programs. Furthermore, such wrong estimation may lead to inappropriate or even harmful diet plans.

Therefore, estimating physiological and anatomical metrics, particularly ambulatory metrics, accurately is important to assess the status of a patient or the wellbeing of a person with high (or at least known) reliability. Accordingly, there is need for these errors to be minimized. In particular, assessing the reliability becomes paramount to guarantee the quality of the estimated physiological metrics.

SUMMARY OF THE INVENTION

It is an object of the present invention to improve the reliability of estimated metrics relating to a physiological state of a user and hence to assess a user's health status more accurately. In particular, it is an object of the present invention to improve the reliability of an estimated metric relating to a walking ability of a user.

In a first aspect of the present invention a method for assessing reliability of an estimated metric relating to a physiological state of a user is presented, the method comprising:

obtaining one or more sensor signals relating to the physiological state of the user;
estimating independently from each other a first metric and a second metric using a different method and/or algorithm based on the one or more sensor signals, wherein the first metric is related to the second metric by a first mathematical function;
determining a first value of the first mathematical function;
comparing said first value to a bound of the first value;

assessing a reliability rating for the estimation of the first metric and/or the second metric based on said comparison; and outputting the assessed reliability rating.

In a further aspect of the present invention a device for assessing reliability of an estimated metric relating to a physiological state of a user is presented, the device comprising:

a sensor input configured to obtain one or more sensor signals relating to the physiological state of the user;
a processor configured to:

estimate independently from each other a first metric and a second metric using a different method and/or algorithm based on the one or more sensor signals, wherein the first metric is related to the second metric by a first mathematical function;
to determine a first value of the first mathematical function;
to compare said first value to a bound of the first value; and
to assess a reliability rating for the estimation of the first metric and/or the second metric based on said comparison; and

an information output configured to output the assessed reliability rating.

In yet another aspect of the present invention a system for assessing reliability of an estimated metric relating to a physiological state of a user is presented, the system comprising:

a sensor configured to measure one or more sensor signals relating to the physiological state of the user;
a device as disclosed herein; and
a user interface configured to issue the reliability rating of the device.

In yet a further aspect of the present invention, there is provided a corresponding computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, computer program and medium have similar and/or identical preferred embodiments as the claimed method, in particular as defined in the dependent claims and as disclosed herein.

Conventional methods used for estimating physiological or anatomical metrics, particularly ambulatory metrics, are not as accurate as required. In particular, estimations deviating considerably from the real values are often not recognized. This applies to many kinds of estimations of physiological and anatomical parameters ranging from acceleration estimations to cardio signal estimations.

The present invention proposes a method wherein the reliability of two or more estimated metrics, related to each other, is estimated based on the biomechanical and physiological boundaries of their relationship. In other words, it is proposed to assess the reliability of the estimation of a physiological (or anatomical) metric, particularly an ambulatory metric, by cross-checking different metrics related to each other based on biomechanical, physiological or anatomical boundaries of their relationships.

For many clinical decision making processes it is crucial to determine when estimations are correct since decisions linked to user well-being should be based on reliable metrics. Furthermore, only if the reliability of a metric (i.e. the output of an estimation algorithm used or, in other words, the estimation of an activity or vital parameter) is known precisely, metrics deviating considerably from the real values can be rejected, i.e. sorted out, and neglected in further considerations. Besides, knowledge about the reliability of estimated metrics may help improve algorithms to obtain even more reliable results.

In the method proposed user activity (and/or vital parameters) are monitored and at least two boundary-related physiological (or anatomical) metrics are calculated (or more precisely estimated) from the signals of a monitoring device. For estimating the metrics one or more methods, particularly algorithms, may be used. Then, it is assessed if one metric respects the boundaries dictated by the other metric(s) (and preferably vice-versa). In case a boundary (or boundaries) is not respected, the corresponding metric(s) may be flagged/labelled in a particular manner so that such unreliable metric(s) can be ignored in further considerations. For example, a metric not respecting a bound of another metric may be labelled as "unreliable" and/or a particular reliability score may be provided for said metric.

To be more precise, a first metric A may be estimated using a method g and a second metric B may be estimated using a method h, wherein g and h are methods (or algorithms) using one or more features of the one or more sensor signals. The feature sets of the functions g and h may be the same or may be different. In particular, parts of the features of each feature set may be the same. Yet, the metrics may generally be estimated independently from each other. The mentioned one or more features may include any of a periodicity of an acceleration signal, a standard deviation of an acceleration signal, a slope of a pressure signal (e.g. from a barometer), an average heart rate, a maximum heart rate and breathing rate range, for example.

The first metric A and the second metric B may be related to each other by a first mathematical function. For example, A and B may be related to each other via a third metric f(A,B) such that f(A,B)=A/B. By inserting the values of the metrics into the function f(A,B) the first value, C, can be determined. Said first value C is then compared to a bound for said value (or more precisely a bound of the possible values of the function) and, based on the outcome of the comparison, it is assessed whether the first metric and/or the second metric are reliable.

The proposed idea originates from the fact that the values of different physiological metrics may be bounded by each other (i.e., a metric may define the minimum and/or maximum of another metric). Instead of defining bounds for each of the metrics based on the maximum and minimum of the first value C, the estimated first value C can be obtained via f(A,B)=A/B and bounded to get the reliability of both metrics A and B.

As an example, the ambulatory metrics walking speed and step rate (i.e. cadence) are related to each other based on step length; walking speed[t] = measured_step_rate[t] * measured_step_length . Furthermore, both metrics are bounded by one another. The metrics, as examples of a first metric and a second metric, respectively, may be extracted from one or more sensor signals. For example, walking speed may be estimated based on a signal provided by an inertial sensor, such as an accelerometer or gyroscope. At the same time, the step rate of a user may be estimated either from the same sensor signal or from another sensor signal (using the same or other signal features). Since the first metric walking speed is related to the second metric step rate by a step length function from the estimations of both metrics a value of the step length function (i.e. the first value) may be calculated. Since the step length represents a bounded value (both bounded from above and below) the estimated (i.e. calculated) step length may be compared to its bound.

In general, comparison of the first value with its bound may include a direct comparison as well as an indirect comparison.

In a direct comparison it may be checked whether the first value respects its bound, i.e. whether the first value is lower, equal or higher than the bound. A direct comparison may also include calculating the difference between the first value and the bound. In the example mentioned above it may be checked whether the estimated value of the step length is smaller than an upper bound for the step length, for example. Likewise, the difference between the upper bound of the step length and the estimated value of the step length may be calculated.

On the other hand, in an indirect comparison the first value may not be compared to the bound directly, but indirectly by comparing it to a function of the bound. The function of the bound may be function of the difference between the bound and the first value, for example. In the above example, a function including the difference between the estimated value of the step length and its bound may be calculated. Furthermore, the result of an evaluation of the function may be compared to a bound.

The results of the comparison may then be used to assess the reliability rating of the estimation regarding the first metric and/or the second metric. The reliability rating may either be output directly or may be filtered before outputting. For example, reliability ratings below (or above) a predefined limit value may be sorted out as unreliable and thus may not be output. On the other hand, only a reliability rating below (or above) a predefined limit value may be output.

In general, there may be determined a plurality of (values of) first metrics and/or second metrics using different methods/algorithms. Accordingly, a plurality of first values of the mathematical function may be determined using these metrics and the plurality of first values may be compared to the bound of the first values. Accordingly, a plurality of reliability ratings for the estimation of the first metric and/or the second metric based on said comparisons may be assessed. The plurality of reliability ratings may then be analysed and the methods used for determining the metrics may be selected in accordance with the reliability ratings. In particular, only those methods for estimating a metric may be used further which have resulted in a reliability rating of the corresponding metric being higher than a predetermined threshold. Hence, the reliability of metric estimations can be improved further.

The main application of the present invention is related to health monitoring in hospital and home, with a particular focus on sleep and respiratory care, vital signs sensing and connected sensing.

In an embodiment of the method comparing the first value to the bound of the first value comprises determining whether the first value fulfils a relationship between the first mathematical function and the bound.

The relationship between the first mathematical function and the bound may comprise an equation or an inequality. Accordingly, it is checked whether the first value fulfils said equation or inequality. For example, the first value may be A/B, wherein A is the first metric and B is the second metric, and it may be checked whether A/B < C, C representing the bound value. In general, the relationship between the first mathematical function may comprise any of a difference between the first mathematical function and the bound, a sum of the first mathematical function and the bound, a fraction of the first mathematical function and the bound and a multiplication of the first mathematical function and the bound.

The bound may be time-dependent or user dependent. User dependency may comprise a dependency on any of age, weight, height, gender, leg length, foot size or any other physiological or anatomical feature of a user.

In an embodiment the bound is a lower bound and/or an upper bound.

For example, the first metric may be walking speed and the second metric may be step rate. Both metrics are related via step length. The value for the step length of a user is bounded by physiological/anatomical restrictions such as the leg length of the user (or people in general). Accordingly, the bound of the first value may be upper bound of the step length. In a similar manner a lower bound for the value of the step length or another first value may be defined.

In another example, the first metric may be the heart rate of a user and the second metric may be activity counts (during a specific activity) of the user. The relationship between these metrics is highly dependent on the fitness level of the user. The fitness can be expressed in terms of VO2max (i.e. the maximum rate of oxygen consumption measured during incremental exercise), which is bounded. The lower and upper bounds of VO2max may be determined based on body characteristics of the user, such as weight, BMI, fat free mass and age, for example.

As an example, the lower bound may be set to zero and the upper bound may be set at (plus) infinity.

In another embodiment comparing the first value to the bound of the first value comprises determining whether the first value is higher than the lower bound and/or lower than the upper bound.

Likewise, it may be determined whether the first value is equal to or lower than the lower bound and/or whether the first value is equal to or higher than the higher bound. For example, the value of the estimated step length may be compared to the lower bound including determining whether the value of the estimated step length is higher than said lower bound.

In another embodiment the method the bound is predetermined or the method further comprises estimating the bound based on any of a previous first metric, a previous second metric, a previous first value or the one or more sensor signals.

The bound, particularly a lower bound and/or an upper bound, may be determined from literature. For instance, as is known from literature, during walking the minimum step length of a person may be 30 cm and the maximum step length may be 90 cm. However, during running these bounds may be different as indicated by literature data.

However, the bound may be determined empirically as well. In particular, the bound may depend on the one or more signals obtained and/or on the estimated metrics based on these signals. In fact, the lower and upper bound for the step length may vary based on an accelerometer signal obtained or based on an estimated metric from it. The bounds may be extrapolated as statistics from recorded data (e.g. for user A 99% of the fraction (time walking speed / step rate) < N, then N may be the upper boundary). In fact, based on empirical knowledge fixed thresholds may be extrapolated by common sense or based on previously collected data.

Furthermore, the bound may depend on one or more predefined parameters.

In any case, particularly for estimated, i.e. empirically determined lower and upper bounds, possible estimation errors may further be considered. Accordingly, if the step length is mainly overestimated, the bounds, particularly the upper bound for the step length can be lowered.

In yet another embodiment of the method any of the first metric, the second metric and the first mathematical function may be time dependent. For example, the relation between the first metric, i.e. the estimated walking speed, and the second metric, i.e. the estimated step rate, may change according to day time or a time elapsed during walking.

Furthermore, any of the first metric, the second metric and the first mathematical function may depend on a user's physiological parameters.

In a further embodiment of the method further comprises determining a second value of a second mathematical function relating the first mathematical function to the bound, wherein assessing the reliability rating for the estimation of the first metric and/or the second metric is based on the second value.

The second mathematical function may particularly include a difference between the bound of the first value and the value of first mathematical function (when the first and second metric are inserted). For example, if the first mathematical function represents the step length the second mathematical function may include a difference between the lower bound MIN_SL of the step length and the value of the estimated step length. Similarly, the second mathematical function may include a difference between the upper bound MAX_SL of the step length and the value of the estimated step length. For example, the second function may be

$$1 - \frac{MIN\_SL(t) - first\ value}{MIN\_SL(t)}$$

or

$$1 - \frac{first\ value - MAX\_SL(t)}{MAX\_SL(t)}.$$

As can be seen from the example, the second mathematical function may be time dependent. Particularly, the second mathematical function may be a function of a time-dependent bound of the first value. However, the second mathematical function may also include a time-dependent first mathematical function. However, the second mathematical function may likewise be time independent.

In another embodiment assessing the reliability rating comprises assigning a score of a binary or continuous scale to the estimation of the first metric and/or the second metric.

For example, the reliability rating of the metrics can be expressed in terms of binary scores "reliable" and "unreliable". For example, in case of the two metrics estimated speed and estimated step rate, the first function may be defined as estimated step length. The first value of the step length may be compared to a minimum step length MIN_SL and a maximum step length MAX_SL. In other words, it may be checked whether the relation

$$MIN\_SL < first\ value < MAX\_SL$$

is fulfilled. If the estimated step length is smaller than the minimum step length and if the estimated step length is higher than the maximum step length the estimated metrics may both be assigned the score "unreliable". In any other case the estimated metrics may be assigned the score "reliable".

On the other hand, if the first value is higher than the higher bound for said value the reliability may be set to be zero and otherwise to one. Furthermore, if the first value is lower than the lower bound for said value the reliability may be set to be zero and otherwise to one, wherein a reliability rating of zero may correspond to an unreliable estimated first value and a reliability rating of one may correspond to a reliable estimated first value.

As another example, there may be determined two second functions, in particular a first second function representing "lower step length reliability",

$$1 - \frac{MIN\_SL(t) - first\ value}{MIN\_SL(t)}$$

and a second second function representing "upper step length reliability"

$$1 - \frac{first\ value - MAX\_SL(t)}{MAX\_SL(t)}.$$

In case the first or second second function is smaller than zero the value of the first or second second function, respectively, may be set to zero. On the other hand, if the first or second second function is higher than one the value of the first or second second function, respectively, may be set to one. The reliability rating may be set to the smaller value of the values of the first second function and the second second function, i.e. the reliability rating may be set to

$$MIN(1 - \frac{MIN\_SL(t) - first\ value}{MIN\_SL(t)}, 1 - \frac{first\ value - MAX\_SL(t)}{MAX\_SL(t)}).$$

Hence, the reliability rating may be represented by a score in the range between zero and one.

In yet another (different) example the reliability rating may be any score between zero and one (or any other numbers) depending on the difference between the first value and the bound, for example. If the difference between the first value and the upper bound and the first value of the lower bound is equal, i.e. if the first value is in the middle between the lower bound and the upper bound, the reliability may be set to one. On the other hand, the score of the reliability rating may decrease the further away the first value is from the lower but the closer to the upper bound and vice versa, wherein if the first value is lower than the lower bound or higher than the upper bound the score of the reliability rating is set to zero, implying that such first values are not reliable.

In yet another alternative example any first value between a lower and an upper bound could be considered reliable and first values being outside any of the bounds may be assigned a reliability rating which is decreasing with increasing distance from the bounds.

In a further embodiment the method further comprises adapting or changing a method for estimating the first metric and/or the second metric based on the reliability rating. In other words, the reliability estimations may be used to update the estimations of the first and/or second metric. In general, more than one estimation method may be used for estimation of one (or both) of the metrics. For example, method A may be used for estimating walking speed, whereas methods A and B may be used to estimate the step rate, or even methods X and Y may be used for speed estimation and methods A and B (and C, etc.) for step rate. In such cases the corresponding reliability ratings could be used to select which predicted, i.e. estimated, metric (e.g. the step rate estimation of algorithms A or B) or combination of estimated metrics (e.g. step rate estimations of algorithm A combined with speed of algorithm X) to be used for further considerations, particularly further reliability assessments. Furthermore, based on the reliability ratings it may be decided which metric may be output directly or used as further input of the health assessment. This way the accuracy of metrics can be improved, while coverage can be kept high as well.

As another example, algorithm A1 may be used for speed estimation resulting in an estimated speed a1, algorithm A2 may be used for speed estimation resulting in an estimated speed a2, algorithm A3 may be used for speed estimation resulting in an estimated speed a3 and so on. Assuming that the algorithms are different from each other not all these algorithms may perform equally well for the various possible walking speed or walking patterns applied to. For example, algorithm A1 may be the most accurate algorithm for low speed, whereas for a higher speed algorithm A2 may be more suitable and for walking with walking aids or the like algorithm A3 may be the best choice. If speed estimation a1 is unreliable (based on the method described above) it should obviously not be used to assess the user. Accordingly, algorithm A1 may be rejected (excluded) for speed estimation. The exclusion of algorithm A1 and hence the exclusion of the estimation a1 decreases the coverage of the speed estimation (i.e. resulting in one data point less). However, this can be overcome by using in parallel other algorithms (A2, A3, ... An). These methods are checked for reliability and the method with the best reliability is used instead of algorithm A1.

Additionally after the exclusion of speed estimation a1, the speed estimation of the other algorithms may be merged (for instance by averaging the speed estimations). In fact, although walking speed and other parameters representing the physiological or anatomical features of a user when measured within and across individuals show similar bounds and relationships among each other and hence typically stay within certain bounds, there are often particular parameters dictating the kinds of relation. Latt, M. D., Menz, H. B., Fung, V. S., & Lord, S. R.: "Walking speed, cadence and step length are selected to optimize the stability of head and pelvis accelerations", Experimental brain research, 184(2), 201-209, discloses a variety of relationships between self-selected gait speed (i.e. gait speed as experienced by a user) and several step related metrics, for example. As can be seen from said document, the relationship between measured speed and step rate is linear up to approximately 2.0 m/s, corresponding to a step rate of approximately 150 steps/min. However, error bars at this point are very large. Indeed, for faster walking speed, the relationship between the two metrics changes. This is due to the fact that at this point the user's activity changes from walking to jogging. The relationship between the two metrics is mediated by several person-dependent biomechanical characteristics and contextual variables. One of the predominant factors is step length variability. In fact, step length is not constant for the same speed between and within people. Because of this, speed estimations via step rate can have a considerable error (even for a speed lower than 2 m/s). This "weak" relationship does not allow to accurately calculate the step rate based on speed (and vice versa) by knowing the step length. However, it allows setting boundaries to each one of the metrics based on the minimum and maximum possible step length values. Furthermore, using the data disclosed the boundaries for the reliability estimations may be adapted. Besides, the algorithms used may be changed in accordance with the value of a previously estimated metric, or more precisely, according to a parameter the metric depend on, or in accordance with the first value.

In yet a further embodiment the one or more sensor signals comprise any of an acceleration signal, a gyroscope signal, a heart rate signal, a respiration signal, an oxygen saturation (SpO2) signal, a motion signal, a force signal, a pressure signal, an electromyography (EMG) signal, a temperature signal, a calorie expenditure signal, a global positioning system signal, a camera signal, a radar signal, a speed measurement signal, a torque measurement signal, an electrocardiography (ECG) signal, an electroencephalography (EEG) signal, a photoplethysmography (PPG) signal, a barometer signal, a muscle tone signal, a sweat level signal, a stress level signal, a skin conductance signal, a galvanic skin response signal, an inertial measurement signal and a blood pressure signal, and/or wherein the first metric and/or the second metric comprise any of walking speed, step rate, step length, heart rate, energy expenditure and activity type.

Any other metric related to a physiological or anatomical state of a user is likewise conceivable.

In an embodiment of the system the sensor comprises any of an accelerometer, a gyroscope, a heart rate sensor, a respiration sensor, an oxygen saturation (SpO2) sensor, a motion sensor, a force sensor, a pressure sensor, an electromyography (EMG) sensor, a temperature sensor, a calorie expenditure sensor, a global positioning system sensor, a camera, a radar, a speed measurement sensor, a torque measurement sensor, an electrocardiography (ECG) sensor, an electroencephalography (EEG) sensor, a photoplethysmography (PPG) sensor, a barometer, a muscle tone sensor, a sweat level sensor, a stress level sensor, a skin conductance sensor, a galvanic skin response sensor, an ineratial measurement sensor and a blood pressure sensor.

In another embodiment of the system the sensor is a remote sensor or a wearable sensor. The sensor may particularly be a contact sensor. Remote sensors include a camera (recording the user's activity), or a shoe sensor, for example. A wearable sensor may be integrated into wearable objects, such as a pendant or a trouser pocket, or may be attached to any of wrist, fingertip, earlobe, in-ear parts, torso and so on, and may include any of a smart phone, a smart watch and an in-ear device, for example.

The system may comprise one or more sensors. In case of a system comprising a plurality of sensors one or more of the sensors may be wearable sensors. Wearable sensors provide high unobstrusiveness and are particualrly convenient for long-term use. In particular, ambulatory metrics may be estimated by using wearable sensors.

BRIEF DESCRIPTION OF THE DRAWINGS

These and other aspects of the invention will be apparent from and elucidated with reference to the embodiment(s) described hereinafter. In the following drawings

Fig. 1 shows a flow chart of an embodiment of a method according to the present invention;
Fig. 2A shows a diagram of a relationship between step length and walking speed as measured;
Fig. 2B shows a diagram of a relationship between step length to height ratio and walking speed as measured;
Fig. 2C shows a diagram of a relationship between step length and walking speed as estimated;
Fig. 2D shows a diagram of a relationship between step length to height ratio and walking speed as estimated;
Fig. 2E shows a diagram of a relationship between estimated walking speed and measured walking speed;
Fig. 2F shows a diagram of a relationship between estimated step rate and measured step rates;
Fig. 3 shows a schematic diagram of an embodiment of a device according to the present invention; and
Fig. 4 shows a schematic diagram of an embodiment of a system according to the present invention.

DETAILED DESCRIPTION OF EMBODIMENTS

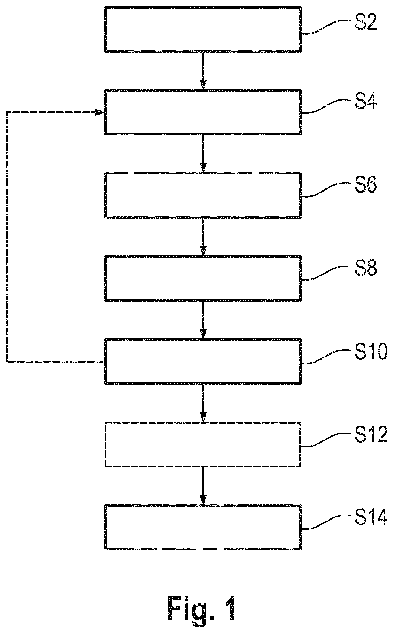

Fig. 1 shows a flow chart of an embodiment of a method according to the present invention. The steps of the method may be carried out by the device 10, wherein the main steps of the method are carried out by the processor 14. The method may e.g. be implemented as computer program running on a computer or processor.
In a first step S2 a plurality of sensor signals are obtained from a plurality of sensors. The sensor signals relate to the physiological state of a user. In particular, the signals relates to the ambulatory capacity of the user. In a second step S4 there are extracted a first metric and a second metric from the signals. In other words, using the sensor signals, there is estimated a first metric and a second metric. In this embodiment the first metric is estimated using an algorithm A and an algorithm B and the second metric is estimated using the algorithm A and the algorithm B. Hence, there are in fact estimated two values of the first metric and two values of the second metric. The first metric and the second metric are chosen such that they are related to each other by a mathematical function the values of which are bounded. In a next step S6 a first value of the mathematical function is derived (calculated) by inserting the first and second metric as estimated by algorithm A. Furthermore, another first value of the mathematical function is derived by inserting the first and second metric as estimated by algorithm B. Then, in step S8, said first values are each compared to a bound for these values. In this embodiment the bound is predetermined. Depending on the result of the comparison there is assessed a reliability rating for each of the estimated first values and hence for the estimation of the first metric(s) and the second metric(s) in step S10. Hence, there are obtained two reliability ratings. One for the metrics based on algorithm A and another for the metrics based on algorithm B. The reliability ratings are binary in this embodiment. Either the first metrics are labelled as "reliable" or they are labelled as "unreliable". The same applies to the second metrics. In an optional step S12 the reliability ratings obtained are filtered with particular ratings being sorted out. To be more precise, the estimated metrics corresponding to an "unreliable" rating are sorted out. In step S14 the assessed reliability rating (and the corresponding metrics) is output, wherein if the "unreliable" metrics have been sorted out in step S12 only "reliable" ratings (and metrics) are output.
The reliability ratings assessed for the first values (and corresponding metrics) in step S10 may (optionally) also be used as input in step S4. In particular, if the reliability rating for the metrics as estimated using algorithm A is higher than the reliability rating for the metrics as estimated using algorithm B an estimation using algorithm B in the future may be omitted.

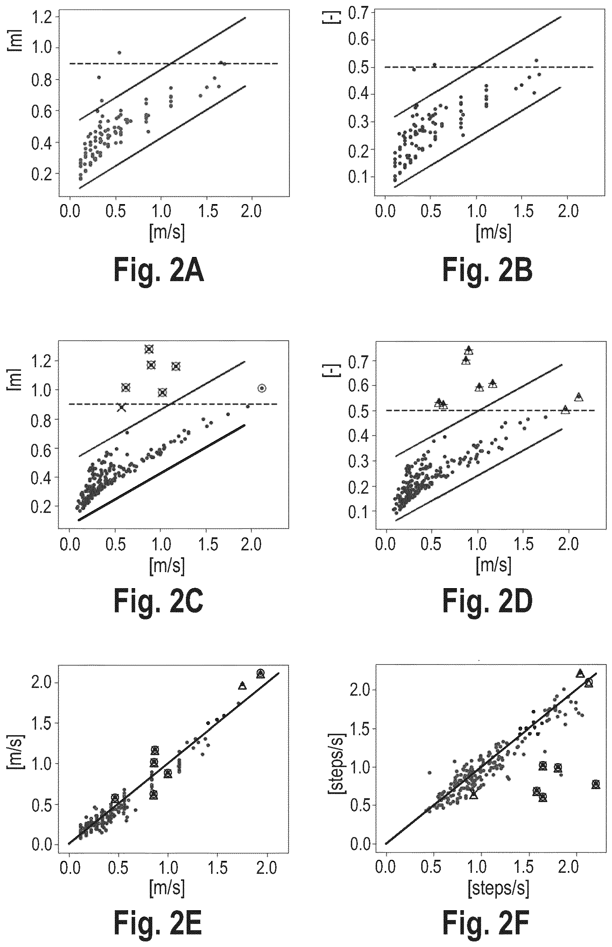

Figs. 2A and 2B show the (actual) relationship between walking speed and step length and walking speed and step length to user height ratio as measured for a first dataset, respectively, wherein by height a user's body height is implied. In other words, the depicted data points are points corresponding to a measurement and are not estimated using an algorithm. In particular, the diagram of Fig. 2A shows on the y-axis the step length in units of meters of a user as measured and on the x-axis the walking speed in units of meters per second of the user as measured. The diagram of Fig. 2B shows on the y-axis the step length to user height ratio as measured and on the x-axis the walking speed of in units of meters per second for the user as measured. From these (reference) measurements likelihood boundaries are determined. The solid lines shown in Figs. 2A and 2B indicate 95% estimation intervals for linear regression, i.e. there is a 95% probability that the true best-fit line for the data points lies within the confidence interval marked by the solid lines. The 95% estimation intervals for linear regression are used as speed/step rate-dependent bounds in this embodiment.
Similarly, the dashed line indicates an upper threshold for the shown data. In particular, as can be seen from Fig. 2A, an upper bound for the absolute estimated step length may be set at 0.9 m (Max_SL = 90 cm). Likewise, an upper bound for the step length to a users's body height ratio may be set at 0.5 (Max_SL / height = 0.5) (see dashed line in Fig. 2B).
Figs. 2C and 2D show the relationship between predicted, i.e. estimated, speed (using an algorithm that takes accelerometer data, i.e. an accelerometer signal, as input) and predicted, i.e. estimated, step length and step length to height ratio (also using an algorithm that takes accelerometer data as input), respectively, for a second dataset (different from the first one used to determine the boundaries). In particular, the diagram of Fig. 2C shows on the y-axis the step length in units of meters of a user as estimated and on the x-axis the walking speed in units of meters per second of the user as estimated. The diagram of Fig. 2B shows on the y-axis the step length to user height ratio as estimated and on the x-axis the walking speed of in units of meters per second for the user as estimated.

The estimations of the step length and the step length to height ratio may be based on the fact that the estimated step length can be expressed as ratio of estimated speed and estimated step rate. The accelerometer used for measurements of walking speed and step length to height ratio in this embodiment is a pendant-worn accelerometer. The bounds shown in Figs. 2C and 2D correspond to those of the measured data shown in Figs. 2A and 2B. As can be seen from Figs. 2C and 2D in comparison with Figs. 2A and 2B, respectively, the estimated values have a similar relationship with each other as the measured ones, aside from some outliers, which can be regarded as an indicator that the algorithms used for estimation of the step length and step length to height ratio (or more precisely the algorithms for walking speed and step rate) work as desired. On the other hand, if the predicted values had a very different relationship with each other than the measured ones, this would be and indicator that at least one of the algorithms used (either the algorithm for walking speed estimation or the algorithm for step rate estimation) would generate incorrect predictions/estimations and would hence be flawed.

In Figs. 2E and 2F the estimated speed and the estimated step rate are plotted against the (reference) speed and step rate, respectively. To be more precise, the diagram of Fig. 2E shows the estimated walking speed in [m/s] (y-axis) in relation to the measured walking speed in [m/s] (x-axis) and the diagram of Fig. 2F shows the estimated step rate in [steps/s] (y-axis) in relation to the measured step rate in [steps/s] (x-axis). The diagrams shown in Figs. 2E and 2F are based on the second dataset.

If the algorithms used for estimation of the walking speed and step rate worked as desired, all data points shown would be on the solid black line. Accordingly, the further away a data point from the black line, the worse the estimations are. Based on the bounds determined in accordance with Figs. 2A and 2B, data points not respecting the bounds are marked as unreliable. In other words, a binary reliability rating approach is applied on walking activity metrics in this embodiment.

For example, in Figs. 2C to 2F data points indicated by an open circle correspond to data points corresponding to a step length that is too large, i.e. outside the upper bound of the step length. Estimated data points corresponding to a too large estimated step length - height ratio are indicated by a triangle. Estimated data points corresponding to a step length estimation being out of its bounds are indicated by a cross and estimated data points corresponding to an estimated step length to height ratio being out of its bounds are indicated by a star. In this way, four step-length-related boundaries can be used to determine unreliable data points.

As can be seen from Figs. 2E and 2F data points marked as unreliable are (in most cases) for the estimated step rate quite far away from the black line and thus are indeed inaccurate, whereas for the estimated walking speed this is less clear. This result is also confirmed by the performance metrics shown in the following table:

|  |  | Speed estimation | | | Step rate estimation | | |
|---|---|---|---|---|---|---|---|
|  | Data kept [%] | MAE [m/s] | RMSE [m/s] | R | MAE [steps/s] | RMSE [steps/s] | R |
| All data | 100% | 0.08 | 0.10 | 0.97 | 0.13 | 0.22 | 0.88 |
| Removing ○ | 97% | 0.07 | 0.09 | 0.97 | 0.11 | 0.15 | 0.93 |
| Removing △ | 96% | 0.07 | 0.09 | 0.97 | 0.11 | 0.15 | 0.93 |
| Removing × | 97% | 0.07 | 0.09 | 0.97 | 0.11 | 0.15 | 0.94 |
| Removing + | 97% | 0.07 | 0.09 | 0.97 | 0.11 | 0.15 | 0.94 |

The table shows the results of speed and step rate estimation if all data are kept (third line) and after removing the data points outside of the imposed respective boundaries (fourth to seventh line). The abbreviation "MAE" stands for "mean absolute error", the abbreviation "RMSE" stands for "root mean square error" and the abbreviation "R" stands for "Pearson's correlation" (here, "R" represents the correlation between measured and predicted/estimated values).

As can be seen from the table the reliability of the metrics shows improvement when excluding data based on estimated step length (ratio), particularly for step rate (see right part of the table). In fact, if all predictions/estimations were exactly correct, all errors shown in the table would be zero and "R would be one. If only random points were removed the accuracy of the data would not have improved that significantly. While the improvement comes at the cost of not estimating an outcome for a certain part of the data, it can be seen that this is effect is relatively limited, with at most 4% (see first column).

While in the above example both estimated metrics, i.e. both the estimated step length and the estimated step rate, are considered unreliable if the first values step length and step length to height ratio are not within their imposed boundaries, there are other embodiments conceivable in which the estimated metric considered most reliable (e.g., based on previously calculated results) can be used as a guideline for the other metric, therefore considering unreliable only the latter if the boundaries are not respected. For instance, in the above example, the speed estimation is on average more accurate than the step rate estimation. Accordingly, only the estimated step rate could have been considered unreliable such that the estimated speed could have been kept. Furthermore, the step rate estimation may be updated based on the speed estimation and the step length and/or step length to height ratio boundaries.

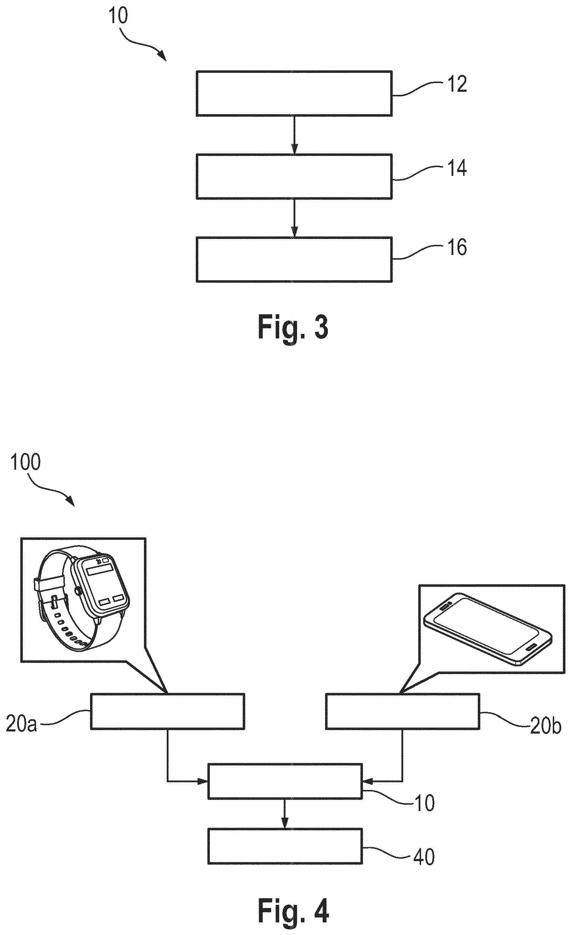

Fig. 3 shows a schematic diagram of an embodiment of a device 10 according to the present invention. The device 10 comprises a sensor input 12, a processor 14 and an information output 16.

The sensor input 12 is configured to obtain at least one sensor signal from at least one sensor, the sensor signal relating to the physiological state of the user. The sensor input 12 may be directly coupled or connected to the sensor(s) or may obtain (i.e. retrieve or receive) the sensor signals from a storage, buffer, network, or bus, etc. The sensor input 12 may thus e.g. be (wired or wireless) communication interfaces or data interfaces, such as a Bluetooth interface, WiFi interface, LAN interface, HDMI interface, direct cable connect, or any other suitable interface allowing signal transfer to the device 10.

The processor 14 applies at least one algorithm to the signal and its features in order to estimate a first metric and a second metric, the metrics being related to each other by a mathematical relation. In particular, the processor 14 is configured to determine a first value of the first mathematical function by inserting the estimated metrics into the first mathematical function. The first value is then compared to a bound for the first value. Based on the result of the comparison the processor 14 assesses a reliability rating for the estimation of the first metric and/or the second metric.

Subsequently, the information output 16 provides the assessed reliability rating for further processing. The information output 16 may generally be any interface that provides the assessed reliability rating, e.g. transmits it (to another device) or provides it for retrieval (by another device; e.g. a smartphone, computer, tablet, etc.). It may thus generally be any (wired or wireless) communication or data interface.

Fig. 4 shows a schematic diagram of an embodiment of a system 100 according to the present invention. The system comprises two sensors 20a and 20b, the device 10 and a user interface 40. Both sensors 20a and 20b are configured to measure at least one sensor signal relating to the physiological state of a user. In this embodiment the sensor 20a is a smart watch configured to be worn by the user and the sensor 20b is a smartphone configured to be worn by the user. Sensor 20a is configured to measure at least one signal relating to the user's heart rate, while sensor 20b is configured to measure a signal relating to activity counts of the user during a specific activity. The device 10 is configured to obtain the signals of both sensors 20a and 20b and to estimate the user's heart rate and activity counts based on these signals. The relationship between the metrics heart rate and activity count is highly dependent on the fitness level of a person. This fitness can be expressed in terms of VO2max, which generally cannot be above or lower than certain boundary values. Hence, using the boundaries of VO2max the device 10 of the system 100 is configured to assign a reliability rating to the estimated metrics heart rate and activity count. The reliability ratings may then be provided to a user interface 40 which is configured to issue the reliability rating of the device 10. The user interface 40 may generally be any means that outputs the reliability rating in visual or audible form, e.g. in text form, as image or diagram, as sound or spoken words, etc. For instance, the user interface 40 may be a display, a loudspeaker, a touchscreen, a computer monitor, the screen of a smartphone or tablet, etc.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Computer-implemented method for assessing reliability of an estimated metric relating to a physiological state of a user, the method comprising:

   obtaining one or more sensor signals relating to the physiological state of the user;
   estimating independently from each other a first metric and a second metric using a different method and/or algorithm based on the one or more sensor signals, wherein the first metric is related to the second metric by a first mathematical function;
determining a first value of the first mathematical function;
comparing said first value to a bound of the first value;
assessing a reliability rating for the estimation of the first metric and/or the second metric based on said comparison; and
outputting the assessed reliability rating.

2. Method as claimed in claim 1,
wherein comparing said first value to the bound of the first value comprises determining whether the first value fulfils a relationship between the first mathematical function and the bound.

3. Method as claimed in claim 1 or 2,
wherein the bound is a lower bound and/or an upper bound.

4. Method as claimed in claim 3,
wherein comparing said first value to the bound of the first value comprises determining whether the first value is higher than the lower bound and/or lower than the upper bound.

5. Method as claimed in any one of the preceding claims,
wherein the bound is predetermined or wherein the method further comprises estimating the bound based on any of a previous first metric, a previous second metric, a previous first value or the one or more sensor signals.

6. Method as claimed in any one of the preceding claims,
wherein any of the first metric, the second metric and the first mathematical function may be time dependent.

7. Method as claimed in any one of the preceding claims,

   further comprising determining a second value of a second mathematical function relating the first mathematical function to the bound,
   wherein assessing the reliability rating for the estimation of the first metric and/or the second metric is based on the second value.

8. Method as claimed in any one of the preceding claims,
wherein assessing the reliability rating comprises assigning a score of a binary or continuous scale to the estimation of the first metric and/or the second metric.

9. Method as claimed in any one of the preceding claims,
further comprising adapting or changing a method for estimating the first metric and/or the second metric based on the reliability rating.

10. Method as claimed in any one of the preceding claims,

    wherein the one or more sensor signals comprise any of an acceleration signal, a gyroscope signal, a heart rate signal, a respiration signal, an oxygen saturation signal, a motion signal, a force signal, a pressure signal, an electromyography signal, a temperature signal, a calorie expenditure signal, a global positioning system signal, a camera signal, a radar signal, a speed measurement signal, a torque measurement signal, an electrocardiography signal, an electroencephalography signal, a photoplethysmography signal, a barometer signal, a muscle tone signal, a sweat level signal, a stress level signal, a skin conductance signal, a galvanic skin response signal, an inertial measurement signal and a blood pressure signal, and/or
    wherein the first metric and/or the second metric comprise any of walking speed, step rate, step length, heart rate, energy expenditure and activity type.

11. Device (10) for assessing reliability of an estimated metric relating to a physiological state of a user, the device comprising:

    a sensor input (12) configured to obtain one or more sensor signals relating to the physiological state of the user;
    a processor (14) configured to:

- estimate independently from each other a first metric and a second metric using a different method and/or algorithm based on the one or more sensor signals, wherein the first metric is related to the second metric by a first mathematical function;
- to determine a first value of the first mathematical function;
- to compare said first value to a bound of the first value; and
- to assess a reliability rating for the estimation of the first metric and/or the second metric based on said comparison; and

an information output (16) configured to output the assessed reliability rating.

12. System (100) for assessing reliability of an estimated metric relating to a physiological state of a user, the system comprising:

a sensor (20) configured to measure one or more sensor signals relating to the physiological state of the user;
a device (10) as claimed in claim 11; and
a user interface (40) configured to issue the reliability rating of the device (10).

13. System as claimed in claim 12,
wherein the sensor (20) comprises any of an accelerometer, a gyroscope, a heart rate sensor, a respiration sensor, an oxygen saturation (SpO2) sensor, a motion sensor, a force sensor, a pressure sensor, an electromyography (EMG) sensor, a temperature sensor, a calorie expenditure sensor, a global positioning system sensor, a camera, a radar, a speed measurement sensor, a torque measurement sensor, an electrocardiography (ECG) sensor, an electroencephalography (EEG) sensor, a photoplethysmography (PPG) sensor, a barometer, a muscle tone sensor, a sweat level sensor, a stress level sensor, a skin conductance sensor, a galvanic skin response sensor, an ineratial measurement sensor and a blood pressure sensor.

14. System as claimed in claim 12 or 13,
wherein the sensor (20) is a remote sensor or a wearable sensor.

15. Computer program comprising program code means for causing a computer to carry out the steps of the method as claimed in claims 1 to 10 when said computer program is carried out on the computer.

**Patentansprüche**

1. Computerimplementiertes Verfahren zum Beurteilen der Zuverlässigkeit einer geschätzten Metrik in Bezug auf einen physiologischen Zustand eines Benutzers, wobei das Verfahren Folgendes umfasst:

Erhalten eines oder mehrerer Sensorsignale in Bezug auf den physiologischen Zustand des Benutzers;
Schätzen einer ersten Metrik und einer zweiten Metrik unabhängig voneinander unter Verwendung eines unterschiedlichen Verfahrens und/oder Algorithmus basierend auf dem einen oder den mehreren Sensorsignalen, wobei die erste Metrik mit der zweiten Metrik durch eine erste mathematische Funktion in Beziehung steht;
Bestimmen eines ersten Wertes der ersten mathematischen Funktion;
Vergleichen des ersten Wertes mit einer Grenze des ersten Wertes;
Beurteilen einer Zuverlässigkeitsbewertung für die Schätzung der ersten Metrik und/oder der zweiten Metrik, die auf diesem Vergleich basiert; und
Ausgeben der beurteilten Zuverlässigkeitsbewertung.

2. Verfahren nach Anspruch 1,
wobei das Vergleichen des ersten Wertes mit der Grenze des ersten Wertes das Bestimmen umfasst, ob der erste Wert eine Beziehung zwischen der ersten mathematischen Funktion und der Grenze erfüllt.

3. Verfahren nach Anspruch 1 oder 2,
wobei die Grenze eine untere Grenze und/oder eine obere Grenze ist.

4. Verfahren nach Anspruch 3,
wobei das Vergleichen des ersten Wertes mit der unteren Grenze des ersten Wertes das Bestimmen umfasst, ob der erste Wert höher als die untere Grenze und/oder niedriger als die obere Grenze ist.

**5.** Verfahren nach einem der vorstehenden Ansprüche,
wobei die Grenze vorbestimmt ist oder wobei das Verfahren weiter das Schätzen der Grenze basierend auf einer beliebigen einer vorherigen ersten Metrik, einer vorherigen zweiten Metrik, eines vorherigen ersten Wertes oder des einen oder der mehreren Sensorsignale umfasst.

**6.** Verfahren nach einem der vorstehenden Ansprüche,
wobei jegliche der ersten Metrik, der zweiten Metrik und der ersten mathematischen Funktion zeitabhängig sein kann.

**7.** Verfahren nach einem der vorstehenden Ansprüche,

weiter umfassend das Bestimmen eines zweiten Wertes einer zweiten mathematischen Funktion, die die erste mathematische Funktion mit der Grenze in Beziehung setzt,
wobei das Beurteilen der Zuverlässigkeitsbewertung für die Schätzung der ersten Metrik und/oder der zweiten Metrik auf dem zweiten Wert basiert ist.

**8.** Verfahren nach einem der vorstehenden Ansprüche,
wobei das Beurteilen der Zuverlässigkeitsbewertung das Zuweisen einer Punktzahl auf einer binären oder kontinuierlichen Skala an die Schätzung der ersten Metrik und/oder der zweiten Metrik umfasst.

**9.** Verfahren nach einem der vorstehenden Ansprüche,
weiter umfassend das Anpassen oder Ändern eines Verfahrens zum Schätzen der ersten Metrik und/oder der zweiten Metrik basierend auf der Zuverlässigkeitsbewertung.

**10.** Verfahren nach einem der vorstehenden Ansprüche,

wobei das eine oder die mehreren Sensorsignale ein beliebiges umfassen von einem Beschleunigungssignal, einem Gyroskopsignal, einem Herzfrequenzsignal, einem Atmungssignal, einem Sauerstoffsättigungssignal, einem Bewegungssignal, einem Kraftsignal, einem Drucksignal, einem Elektromyographiesignal, einem Temperatursignal, einem Kalorienverbrauchssignal, einem Signal des globalen Positionierungssystems, einem Kamerasignal, einem Radarsignal, einem Geschwindigkeitsmessungssignal, einem Drehmomentmessungssignal, einem Elektrokardiographiesignal, einem Elektroenzephalographiesignal, einem Photoplethysmographiesignal, einem Barometersignal, einem Muskeltonussignal, einem Schweißniveausignal, einem Stressniveausignal, einem Hautleitfähigkeitssignal, einem Signal der galvanischen Hautreaktion, einem Trägheitsmessungssignal und einem Blutdrucksignal, und/oder
wobei die erste Metrik und/oder die zweite Metrik ein beliebiges umfasst von Gehgeschwindigkeit, Schrittfrequenz, Schrittlänge, Herzfrequenz, Energieverbrauch und Aktivitätsart.

**11.** Vorrichtung (10) zum Beurteilen der Zuverlässigkeit einer geschätzten Metrik in Bezug auf einen physiologischen Zustand eines Benutzers, wobei die Vorrichtung Folgendes umfasst:

einen Sensoreingang (12), der konfiguriert ist, um ein oder mehrere Sensorsignale in Bezug auf den physiologischen Zustand des Benutzers zu erhalten;
einen Prozessor (14), der konfiguriert ist zum:

- Schätzen einer ersten Metrik und einer zweiten Metrik unabhängig voneinander unter Verwendung eines unterschiedlichen Verfahrens und/oder Algorithmus basierend auf dem einen oder den mehreren Sensorsignalen, wobei die erste Metrik mit der zweiten Metrik durch eine erste mathematische Funktion in Beziehung steht;
- Bestimmen eines ersten Wertes der ersten mathematischen Funktion;
- Vergleichen des ersten Wertes mit einer Grenze des ersten Wertes;
- Beurteilen einer Zuverlässigkeitsbewertung für die Schätzung der ersten Metrik und/oder der zweiten Metrik, die auf diesem Vergleich basiert; und

eine Informationsausgabe (16), die konfiguriert ist, um die beurteilte Zuverlässigkeitsbewertung auszugeben.

**12.** System (100) zum Beurteilen der Zuverlässigkeit einer geschätzten Metrik in Bezug auf einen physiologischen Zustand eines Benutzers, wobei das System Folgendes umfasst:

einen Sensor (20), der konfiguriert ist, um ein oder mehrere Sensorsignale in Bezug auf den physiologischen Zustand des Benutzers zu messen;
eine Vorrichtung (10) nach Anspruch 11; und
eine Benutzerschnittstelle (40), die konfiguriert ist, um die Zuverlässigkeitsbewertung der Vorrichtung (10) herauszugeben.

13. System nach Anspruch 12,
wobei der Sensor (20) einen beliebigen umfasst von einem Beschleunigungsmesser, einem Gyroskop, einem Herzfrequenzsensor, einem Atmungssensor, einem Sauerstoffsättigungssensor (SpO2-Sensor), einem Bewegungssensor, einem Kraftsensor, einem Drucksensor, einem Elektromyographiesensor (EMG-Sensor), einem Temperatursensor, einem Kalorienverbrauchssensor, einem Sensor des globalen Positionierungssystems, einer Kamera, einem Radar, einem Geschwindigkeitsmessungssensor, einem Drehmomentmessungssensor, einem Elektrokardiographiesensor (EKG-Sensor), einem Elektroenzephalographiesensor (EEG-Sensor), einem Photoplethysmographiesensor (PPG-Sensor), einem Barometer, einem Muskeltonussensor, einem Schweißniveausensor, einem Stressniveausensor, einem Hautleitfähigkeitssensor, einem Sensor der galvanischen Hautreaktion, einem Trägheitsmessungssensor und einem Blutdrucksensor.

14. System nach Anspruch 12 oder 13,
wobei der Sensor (20) ein Fernsensor oder ein tragbarer Sensor ist.

15. Computerprogramm, das Programmcodemittel umfasst, um einen Computer zu veranlassen, die Schritte des Verfahrens nach Ansprüchen 1 bis 10 auszuführen, wenn das Computerprogramm auf dem Computer ausgeführt wird.

**Revendications**

1. Procédé mis en œuvre par ordinateur pour l'évaluation de la fiabilité d'une métrique estimée relative à un état physiologique d'un utilisateur, le procédé comprenant :

l'obtention d'un ou plusieurs signaux de capteur relatifs à l'état physiologique de l'utilisateur ;
l'estimation indépendamment l'une de l'autre d'une première métrique et d'une seconde métrique en utilisant un procédé et/ou algorithme différent sur la base d'un ou plusieurs signaux de capteur, dans lequel la première métrique est liée à la seconde métrique par une première fonction mathématique ;
la détermination d'une première valeur de la première fonction mathématique ;
la comparaison de ladite première valeur à une limite de la première valeur ;
l'évaluation d'une cote de fiabilité pour l'estimation de la première métrique et/ou de la seconde métrique sur la base ladite comparaison ; et
la délivrance en sortie de la cote de fiabilité évaluée.

2. Procédé selon la revendication 1,
dans lequel la comparaison de ladite première valeur à la limite de la première valeur comprend la détermination si la première valeur remplit une relation entre la première fonction mathématique et la limite.

3. Procédé selon la revendication 1 ou 2,
dans lequel la limite est une limite inférieure et/ou une limite supérieure.

4. Procédé selon la revendication 3,
dans lequel la comparaison de ladite première valeur à la limite de la première valeur comprend la détermination si la première valeur est supérieure à la limite inférieure et/ou inférieure à la limite supérieure.

5. Procédé selon l'une quelconque des revendications précédentes,
dans lequel la limite est prédéterminée ou dans lequel le procédé comprend en outre l'estimation de la limite sur la base de l'une quelconque parmi une première métrique précédente, une seconde métrique précédente, une première valeur précédente ou les un ou plusieurs signaux de capteur.

6. Procédé selon l'une quelconque des revendications précédentes,
dans lequel l'une quelconque parmi la première métrique, la seconde métrique et de la première fonction mathématique peut dépendre du temps.

7. Procédé selon l'une quelconque des revendications précédentes,

comprenant en outre la détermination d'une seconde valeur d'une seconde fonction mathématique reliant la première fonction mathématique à la limite,
dans lequel l'évaluation de la cote de fiabilité pour l'estimation de la première métrique et/ou la seconde métrique est basée sur la seconde valeur.

8. Procédé selon l'une quelconque des revendications précédentes,
dans lequel l'évaluation de la cote de fiabilité comprend l'attribution d'un score, sur une échelle binaire ou continue, à l'estimation de la première métrique et/ou la seconde métrique.

9. Procédé selon l'une quelconque des revendications précédentes,
comprenant en outre l'adaptation ou la modification d'un procédé d'estimation de la première métrique et/ou de la seconde métrique sur la base de la cote de fiabilité.

10. Procédé selon l'une quelconque des revendications précédentes,

dans lequel les un ou plusieurs signaux de capteur comprennent un signal d'accélération, un signal gyroscopique, un signal de fréquence cardiaque, un signal respiratoire, un signal de saturation en oxygène, un signal de mouvement, un signal de force, un signal de pression, un signal d'électromyographie, un signal de température, un signal de dépense calorique, un signal de système de positionnement global, un signal de caméra, un signal radar, un signal de mesure de vitesse, un signal de mesure de couple, un signal d'électrocardiographie, un signal d'électroencéphalographie, un signal de photopléthysmographie, un signal barométrique, un signal de tonus musculaire, un signal de niveau de sudation, un signal de niveau de stress, un signal de conductance cutanée, un signal de réponse galvanique cutanée, un signal de mesure inertielle et un signal de pression artérielle, et/ou dans lequel la première métrique et/ou la seconde métrique comprend une vitesse de marche, une cadence de pas, une longueur de pas, une fréquence cardiaque, une dépense énergétique et un type d'activité.

11. Dispositif (10) d'évaluation de la fiabilité d'une métrique estimée relative à un état physiologique d'un utilisateur, le dispositif comprenant :

une entrée de capteur (12) configurée pour obtenir un ou plusieurs signaux de capteur relatifs à l'état physiologique de l'utilisateur ;
un processeur (14) configuré pour :

- estimer indépendamment l'une de l'autre une première métrique et une seconde métrique en utilisant un procédé et/ou algorithme différent sur la base d'un ou plusieurs signaux de capteur, dans lequel la première métrique est liée à la seconde métrique par une première fonction mathématique ;
- déterminer une première valeur de la première fonction mathématique;
- comparer ladite première valeur à une limite de la première valeur ; et
- évaluer une cote de fiabilité pour l'estimation de la première métrique et/ou de la seconde métrique sur la base de ladite comparaison ; et

une sortie d'information (16) configurée pour délivrer en sortie la cote de fiabilité évaluée.

12. Système (100) d'évaluation de la fiabilité d'une métrique estimée relative à un état physiologique d'un utilisateur, le système comprenant :

un capteur (20) configuré pour mesurer un ou plusieurs signaux de capteur relatifs à l'état physiologique de l'utilisateur;
un dispositif (10) selon la revendication 11 ; et
une interface utilisateur (40) configurée pour émettre la cote de fiabilité du dispositif (10).

13. Système selon la revendication 12,
dans lequel le capteur (20) comprend un accéléromètre, un gyroscope, un capteur de fréquence cardiaque, un capteur de respiration, un capteur de saturation en oxygène (SpO2), un capteur de mouvement, un capteur de force, un capteur de pression, un capteur d'électromyographie (EMG), un capteur de température, un capteur de dépense calorique, un capteur de système de positionnement global, une caméra, un radar, un capteur de mesure de vitesse, un capteur de métrique de couple, un capteur d'électrocardiographie (ECG), un capteur d'électroencéphalographie (EEG), un capteur de photopléthysmographie (PPG), un baromètre, un capteur de tonus musculaire, un capteur de niveau de transpiration, un capteur de niveau de stress, un capteur de conductance cutanée, un capteur de réponse galvanique cutanée, un capteur de mesure inertielle et un capteur de pression artérielle.

14. Système selon la revendication 12 ou 13,
dans lequel le capteur (20) est un capteur distant ou un capteur portable.

15. Programme informatique comprenant des moyens de code de programme pour amener un ordinateur à réaliser les étapes du procédé selon les revendications 1 à 10, lorsque ledit programme informatique est exécuté sur l'ordinateur.

Fig. 1

Fig. 2A

Fig. 2B

Fig. 2C

Fig. 2D

Fig. 2E

Fig. 2F

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20130138007 A1
- US 20150099945 A1
- US 20130080255 A1

### Non-patent literature cited in the description

- **LATT, M. D.** ; **MENZ, H. B.** ; **FUNG, V. S.** ; **LORD, S. R.** Walking speed, cadence and step length are selected to optimize the stability of head and pelvis accelerations. *Experimental brain research*, vol. 184 (2), 201-209